# EUROPEAN PATENT SPECIFICATION

(11) **EP 3 076 905 B1**
(45) Date of publication and mention of the grant of the patent: **03.10.2018**
(21) Application number: 14830601.2
(22) Date of filing: 05.12.2014
(51) Int. Cl.: A61F 2/46, A61F 2/34, A61F 2/36, A61F 2/30

(54) **DEVICE FOR EVALUATING CHARACTERISTICS OF A PATIENT FOR SUITABLY APPLYING THERETO A PROSTHESIS**
VORRICHTUNG ZUR BEURTEILUNG DER EIGENSCHAFTEN EINES PATIENTEN ZUR ANWENDUNG EINER PASSENDEN THERAPIE FÜR EINE PROTHESE
DISPOSITIF POUR ÉVALUER DES CARACTÉRISTIQUES D'UN PATIENT POUR LUI APPLIQUER DE FAÇON APPROPRIÉE UNE PROTHÈSE

(30) Priority: 05.12.2013 IT VR20130271
(43) Date of publication of application: 12.10.2016
(73) Proprietor: Aeron S.r.l., 35134 Padova (IT)
(72) Inventor: GARRO, Carlo, I-35010 Campodoro (Padova) (IT); GARRO, Damiano, I-35010 Campodoro (Padova) (IT)
(74) Representative: Feltrinelli, Secondo Andrea
(86) International application number: PCT/IB2014/066642
(87) International publication number: WO 2015/083132

(56) References cited:
- EP-A2- 0 807 426
- WO-A1-01/19296
- WO-A1-2009/108683
- AT-U1- 12 124
- US-B1- 7 425 214

## Description

### TECHNICAL FIELD OF THE INVENTION

The present invention relates to a device for the detection and measurement of physical/structural characteristics of a patient to whom a hip prosthesis must be prepared, more particularly, a device for measuring the femoral anteversion angle, the angle of anteversion of the acetabulum and/or of the cotyle and/or acetabular and or cotyloid inclination angle.

### DESCRIPTION OF THE PRIOR ART

Hip femoral prosthetic systems can present different configurations, but they are all arranged in such a way as to position the centre of joint rotation in a position as anatomical as possible for the respective patient.

In implementing a hip prosthesis, among the various purposes that one should set oneself and that a surgeon must achieve, there is, of course, to restore the limb length, joint stability, as well as the movement range and muscle balance, and this by restoring the horizontal offset or, in other words, reconstructing as faithfully as possible the original anatomical parameters, which may have been altered, for example, due to the onset and development of arthrosis, arthritis or due to trauma suffered by the patient or for other conditions. As it is known, by offset it is intended the distance between the centre of rotation of the joint head and the extension line of the diaphyseal or anatomical axis of the femur.

The femoral prosthetic components may have different configurations, thus each having a respective neck-shaft angle of the so-called "neck" component, standard or so-called "high offset" stems, necks of different lengths, joint heads with different lengths and diameters, and so on. Even the cotyle or the so-called "insert" can be of different types and configurations from one device to another, for example with or without positionable anti-dislocation, inclined, eccentric, protruded, retentive, "shoulders" or "roofs" etc., so that adaptation can be obtained for different situations and morphologies.

During surgery, the surgeon, whether (s)he has made or not a preoperative plan, will test the various options available to ensure that the basic parameters are guaranteed for the patient, including stability, leg length, range of displacement or "range of motion" and muscular balance.

These tests are usually performed through several repeated reduction and dislocation manoeuvres of the joint, by inserting a "femoral trial rasp" or trial rasp into the femur and then changing on the femoral trial rasp the various available trial necks and combining with these the various lengths of trial joint heads; every configuration change and check inevitably involves a joint dislocation and reduction manoeuvre, resulting in wasted time, increased surgical exposure times and increased surgical damage to the joint itself and to the muscles involved. On the basis of the outcome of the performed tests, the surgeon will then choose the final implant configuration.

This procedure is applied in combination with every intervention or surgical access way, as well as for any type of prosthesis, for both traditional techniques and techniques involving the use of "navigators" or robots and for minimally invasive techniques; in the latter case, in particular, the purpose is to minimise the surgical joint damage, in order to obtain a quicker postoperative functional recovery that is easier for the patient.

AT12124U1, EP0807426A2 and US7425214B1 disclose devices according to the state of the art.

### OBJECTS OF THE INVENTION

The main object of the present invention is to provide a new device for the detection and, optionally, the measurement, in the course of the implementation of a hip prosthesis, of the physical/structural characteristics of a patient to whom a hip prosthesis must be made.

Another object of the present invention is to provide a new measuring device for measuring the angle of the femoral anteversion, the angle of anteversion of the acetabulum and/or of the cotyle and/or the angle of the acetabular and/or the cotyloid inclination for making a hip prosthesis.

In connection with an aspect of the invention a device is provided according to claim 1.

In connection with an aspect of the invention a kit is provided according to claim 16.

Dependent claims refer to preferred and advantageous embodiments of the invention.

### BRIEF DESCRIPTION OF THE DRAWINGS

Other characteristics and advantages of the invention will be more apparent from the description of embodiments of devices, illustrated only by way of example in the accompanying drawings in which:
- Figure 1 is a side view with sectional parts of a device not forming part of the present invention;
- Figure 2 is a view similar to Fig. 1 of another embodiment of a device not forming part of the present invention;
- Figure 3 is a view similar to Fig. 1 of a further embodiment of a device not forming part of the present invention;
- Figure 4 is a comparative schematic view of different operating positions of the device of Fig. 3;
- Figures 5 and 6 are front views in respective operating positions of another example of a device not forming part of the present invention;
- Figures 7, 8 are perspective views slightly from above and from one side of the device of Figures 5 and 6;
- Figure 9 is a perspective view slightly from above and from another side of the device of Figures 5 and 6;
- Figure 10 is a side view of the device of Figures 5 and 6;
- Figure 11 is a perspective view slightly from above of a device according to the present invention;
- Figure 12 is a view from above of the device of Fig. 11;
- Figure 13 is a view similar to Fig. 12 with parts shown in transparency;
- Figure 14 is a side view with parts in transparency of the device of Fig. 11;
- Figure 15 is an exploded view of the device of Fig. 11;
- Figures 16 and 17 are front and side views, respectively, of a kit according to the present invention during one assembly phase;
- Figure 18 is a front view with parts shown in transparency of a kit according to the present invention during one assembly phase;
- Figures 19 and 21 are front and side schematic views, respectively, of a femur in which a device is inserted of a kit according to the present invention; and
- Figures 20 and 22 are front and top schematic views, respectively, of a pelvis.

In the accompanying drawings the same parts or components are indicated by the same reference numerals.

### EMBODIMENTS OF THE INVENTION

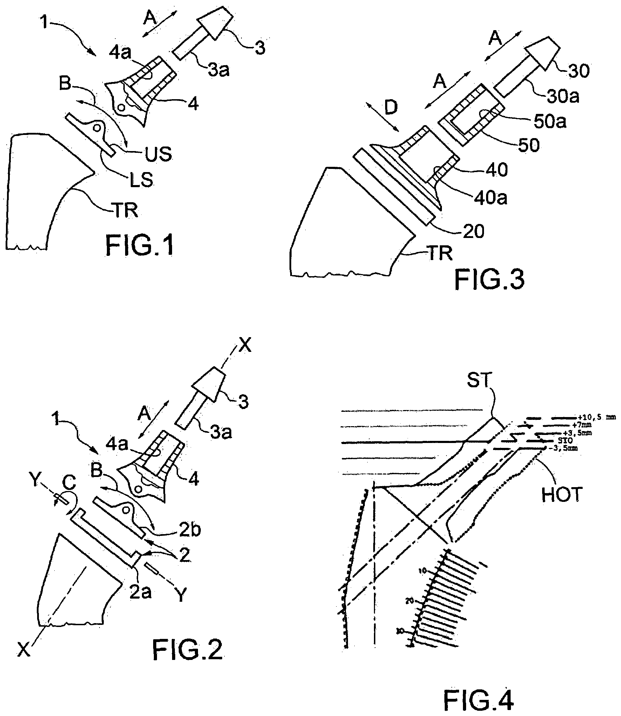

With reference to Figures 1 to 4, there is shown a trial neck device 1 of a kit not forming part of the present invention, which device is for intraoperative measurement of the physical/structural characteristics of a patient for the realization of a hip prosthesis and includes a base component 2 intended to be constrained to a trial rasp TR or to a final modular femoral prosthetic component (i.e. the one either inserted or to be inserted and fixed in the femur of the patient) and a terminal or apical component 3 constrained to the base component 2 and intended to be housed proximally to or in the vicinity of the cotyle of a patient, for example, within the so-called head or small-head component to be inserted into the cotyle or in the so-called "insert" component.

Adjustment means are then provided for adjusting the distance (see arrow A in the Figure) and/or the angular position (see arrow B in the Figure) of the apical component 3 with respect to the base component 2. The apical component 3 or rather the side surface of same can be, for example, configured as a cone or truncated cone, with a tapered cross section in the moving-away from the base component 2 direction.

More particularly, the apical component 3 is slidingly mounted with respect to the base component 2 so as to be moveable apart and closer with respect to it. In this regard, the apical component 3 can have a stem portion 3a intended to slidingly engage in a seat 4a of an intermediate group, comprising a first intermediate component 4, which intermediate group 4, on the one side, is constrained to the base component 2 and, on the other side, to the apical component 3. The stem portion 3a can, according to a variant, be slidingly mounted within a seat formed in the base component 2. The apical component 3 can be locked at different operating positions with respect to the base component 2 or intermediate component 4.

Alternatively, the first intermediate component 4 may have a stem or shank for sliding engagement with a respective seat of the apical component 3. Furthermore, the first intermediate component 4a could be slidingly engaged with the base component 2.

According to the example illustrated in Figure 1, the intermediate group 4 has at least one portion pivoted to the base component 2, preferably about an axis intended to be substantially parallel to an antero-posterior direction (that is to say, from the front to the rear of the patient treated) so as to assume different neck-shaft angles. Alternatively, the intermediate group 4 could be pivoted to the apical component 3.

Basically, the device 1 has a base body 2, for example substantially flat, provided with a lower surface, in use, LS engageable with the trial rasp TR or with a final prosthetic component, and an upper surface, in use, US, and is hinged, preferably at the upper surface US, to the intermediate component 4. The intermediate component 4 defines instead the seat 4a with main extension, in use, towards the cotyle in which the stem portion 3a of the apical component 3 is slidingly engaged. Naturally, the hinge axis or axes of the intermediate component 4 to the base component 2 is/are substantially orthogonal to the main extension axis of the seat 4a or rather of the stem 3a of the apical component 3.

A device such as that illustrated in Figure 1 can provide, for example, different neck-shaft angles in addition to the ability to change the distance of the apical component 3 from the base component 2.

With reference now to Figure 2, a device similar to that illustrated in Figure 1 is shown, but in which the base component 2 has a first portion 2a intended to be adapted and fixed to a trial rasp TR or to a final prosthetic component, as well as a second portion 2b, preferably hinged or pivoted in the lateral-medial direction (see arrow C) to the first portion 2a. More particularly, the second portion 2b is pivoted to the first portion 2a about an axis y-y orthogonal to both the direction of displacement x-x of the apical component 3, and the axis of rotation (axis entering the sheet) of the intermediate group with respect to the base component 2, or better still, with respect to the second portion 2b.

The second portion 2b, together with the first intermediate element 4 and the apical component 3 constrained to it, can thus assume different inclinations in the antero-posterior direction, in use, which will enable to arrange the elements 3 and 4 with an anteverted, in axis or retroverted orientation. In this regard, in modular neck prostheses, to correct any positioning errors of the femoral stem, if the stem is excessively anteverted, retroverted modular necks are provided and, if the stem is excessively retroverted, anteverted necks are provided.

The device according to Figure 2 can therefore be disposed in different trims, with different neck-shaft angles (see arrow B) and different neck lengths (see arrow A) and also different orientations of the neck device in the antero-posterior, i.e. anteverted, in axis and retroverted directions (see arrow C). To modulate the offset and/or length of the neck device, the length of the apical component 3 can be changed or the length of the prosthetic neck can be changed while maintaining a fixed length of the apical component.

Referring now to Figure 3, a trial neck device 1 is illustrated for the measurement of the physical/structural characteristics of a patient for the realization of a prosthesis comprising a base component 20 adaptable and fixable to a trial rasp TR or to a final prosthetic component, as well as an apical component 30 and an intermediate group comprising one or more, preferably two intermediate components, a first intermediate component 40 constrained to the base component 20 and a second intermediate component 50 constrained, on the one side, to the first intermediate component 40 and, on the other side, to the apical component 30.

More particularly, the base component 20 may have a slide portion, while the first intermediate component 40 has a sliding block portion intended to slidingly engage in the slide portion of the base component 20, so as to allow a shift in the medial-lateral direction, see arrow D in Figure, of the intermediate component 40 with respect to the base component 20.

The second intermediate component 50 may be slidingly mounted with respect to the first intermediate component 40 in the axial direction x-x, for example within a seat 40a delimited by the first intermediate component 40, so as to enable to lengthen or shorten the distance of the apical component 30 (of the free end of apical component) from the base component 20. In this regard, cam means can be provided interceptable by the first intermediate component 40 during its medial shift, so as to determine the displacement of the second intermediate component 50 in the axial direction.

To return the centre of rotation to 0, the apical component 30, according to such embodiment, can be slidingly mounted with respect to the second intermediate component 50 so as to assume the position of the centres of rotation provided for the different lengths of the joint heads. By 0 it is intended the initial position of the device. In this regard, the apical component 30 may have a stem 30a slidingly mounted in a seat 50a delimited by the second intermediate component 50.

This device, as is shown in Fig. 4, can therefore be arranged with a standard ST, or offset or so-called "high offset" HOT or lateralized trim, in addition, of course, to the possibility of variation of the length of the joint heads.

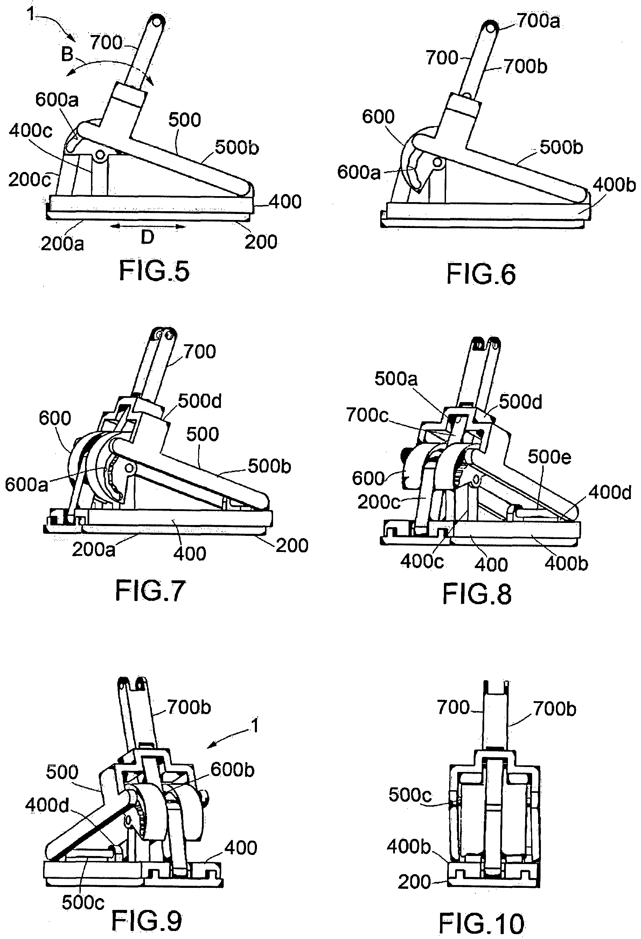

With reference now to Figures 5 to 10, another trial neck device 1 is illustrated for a kit not forming part of the present invention comprising a base component 200 having, for example, a slide portion, while the first intermediate component 400 has, for example, a sliding block portion intended to slidingly engage in the slide portion of the base component 200, so as to allow sliding in the medial-lateral direction, see arrow D in Figure 5, of the first intermediate component 400 with respect to the base component 200.

A second intermediate component 500 is then provided, pivoted or pivotable with respect to the first intermediate component 400 about an axis, antero-posterior in use, so that the device according to Figures 5 to 10 can be arranged in different trims, with different neck-shaft angles (see arrow B).

More particularly, the first intermediate component 400 can have one or a pair of sliding block portions 400b, starting from one or each of which one or more respective stems or upright elements 400c extend, in use, either upwards or towards the cotyle. At the end of the upright elements 400c, distally from the respective sliding block 400b, one or more cam or desmodromic cam components 600 are pivoted. The cam component/s can delimits/delimit a suitably shaped seat 600a, for example extending substantially along a section or length of the circle with centre in the pivoting point of the cam component 600 relative to the respective upright element 400c, said seat 600a being however delimited by a pair of walls having portions or sections substantially inclined with respect to each other.

The second intermediate component 500 comprises instead one or more rod-like elements 500b pivoted, at one end, to the first intermediate component 400, for example to a respective sliding block 400b or to a lug element 400d fixed to it. The other end of the second intermediate component 500 is operatively connected to the cam component 600, for example starting from the other end of the second intermediate component 500 a small pin 500c extends, slidingly mounted within the or a respective seat 600a. Preferably, the second intermediate component 500 has two rod-like elements 500b connected by a bridge-type bracket 500d, optionally substantially U-shaped or with an intermediate U-shaped section, one end of the rod-like elements 500b being connected via a respective pin element 500e. The pin element 500e is pivoted to the first intermediate component 400, for example slidingly mounted within seats formed in the sliding blocks 400b or in lugs 400d extending from the same. Starting from the other end of the rod-like elements 500b, instead, the small pin 500c extends, slidingly mounted within a seat of one or more cam components 600. Naturally, another kind of kinematic connection could be envisaged, for example one or more rod-like elements could delimit a seat engageable by a respective pawl of the cam components.

The base component 200 may instead have a connecting rod or link element 200c, on one side, pivoted to a first lower portion, in use, 200a of the base component 200 and, at the other end, operatively connected to the cam component 600, for example having one or more pawls or cam follower rolls, each slidingly mounted in a respective seat 600a. Naturally, in this case, too, another type of kinematic connection could be provided.

In this device too, then, the apical component (not shown in the drawings) can be slidingly mounted with respect to the base component 200 so as to be movable apart and closer with respect to it. In this regard, the apical component will be fixed or connected to a third intermediate component 700, for example at its free end 700a, optionally fork-like. The third component 700 may have a top portion, in use, 700b, and a stem lower portion, in use, 700c, pivoted to the top portion 700b designed to slidingly engage in a seat 500a of an intermediate group, more particularly of the second intermediate component 500.

The lower stem portion 700c of the third intermediate component 700 is then pivoted to the cam components, for example a sleeve or shank 600b integral with the cam components 600, for example connecting the cam components 600.

In such neck device, by moving an element or component, a corresponding displacement is determined of other elements or components, taking into account that moving, for example, the cam components 600 a displacement is determined of the second and third intermediate component and, of course, of the apical component, while by moving, for example, the sliding block portions 400b of the second intermediate component 400 with respect to the base component 200, a displacement is determined of the small pins 500c and hence, of the cam components 600.

A neck device is preferably configured substantially as the upper part of the femoral bone.

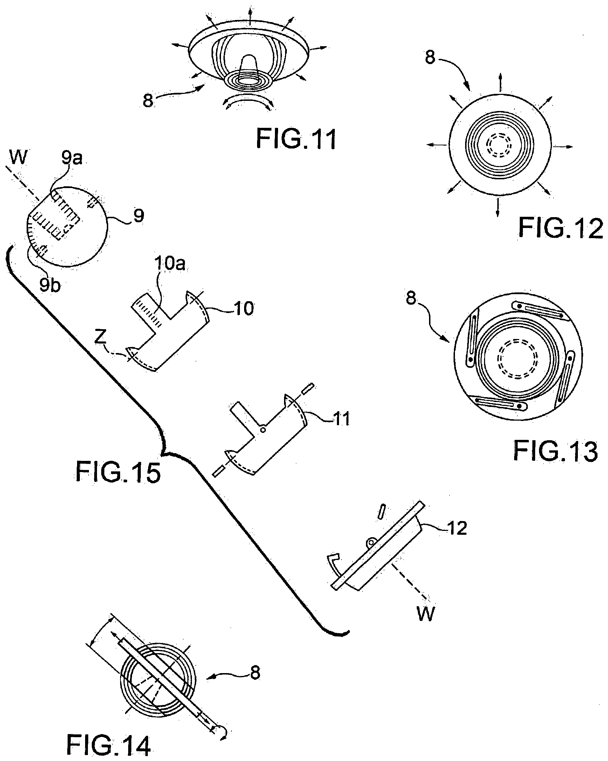

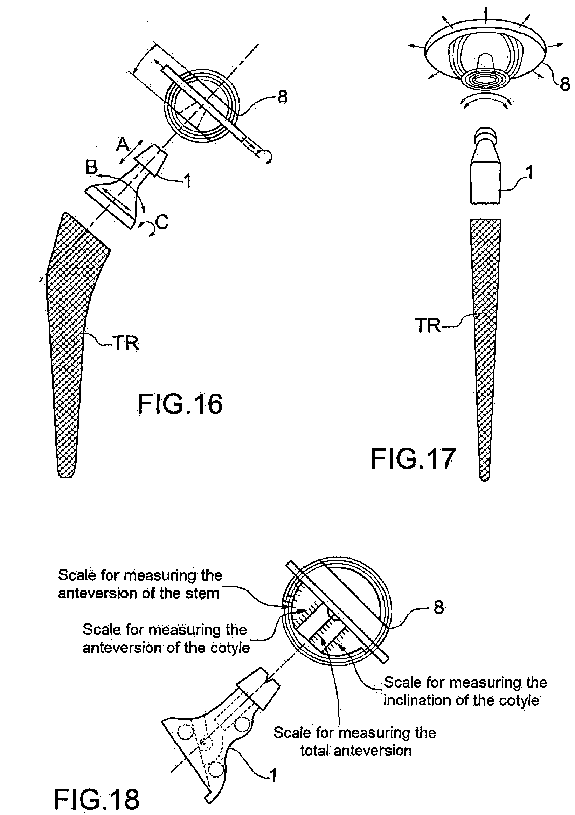

With reference now to Figures 11 to 22, a device 8 is illustrated for measuring the angle of femoral anteversion, the angle of anteversion of the acetabulum and or of the cotyle and/or of the acetabular and/or cotyloid inclination angle for the realization of a hip prosthesis according to the present invention, comprising at least a base or main body 9 mountable on an apical component of a trial neck device, for example on an apical component 3, 30 of a device 1 like the one described above, in this regard see Figures 16 to 18, in which a kit is illustrated according to the present invention comprising a device 1 and 8 during a respective phase of assembly.

The device 8 further comprises at least one auxiliary component engageable with the main component 9, as well as engagement means of the main component with the apical component 3, 30 along a longitudinal axis w-w, for example defining a housing seat 9a of the apical component 3, 30 extending about a longitudinal axis w-w.

The auxiliary component/s is/are displaceable with respect to the main component 9 about a transverse axis, for example orthogonal z-z to the longitudinal axis w-w and, preferably, means are then provided for measuring the displacement of the auxiliary component/s with respect to the base body.

The transverse axis z-z is designed to be located:
- on the front plane and in the inferior medial-lateral upper direction so as to measure the angle of anteversion of the femoral trial rasp or the implanted prosthetic femoral component or the acetabular and/or cotyle anteversion angle; and/or
- on the sagittal plane and in the antero-posterior direction, so as to measure the acetabular and/or cotyloid inclination angle.

More particularly, the base body may comprise a substantially curved outer surface, even more preferably, it can be configured as a sphere or part of a sphere, while the auxiliary component can be configured as a dome or shell mountable on and at least partially around the base body 9.

Each prosthetic cotyloid system of the hip provides different configurations of both the cotyle and the insert with the purpose of ensuring suitable angular ratios between the components such as to ensure the so-called "Safe Zone", the zone within which the centre of rotation of the femoral head of the patient must fall in order to ensure stability.

The device 8 is able to measure all the angles necessary to ensure compliance with the Safe Zone both working in conjunction with the device 1, and on a traditional trial neck or on any prosthetic femoral component, particularly where the latter present an apical component corresponding to a cone, for example the so-called "Morse cone 12-14", usually universally adopted, although, obviously, the device 8 can be adapted in the construction phase to Morse cones of different types.

With particular reference to the embodiment illustrated in Figures, the device 8 comprises a main component 9 configured as a sphere or part of a sphere and one or more, preferably three auxiliary portions 10, 11 and 12 configured as a shell or dome, for example spherical or in any case arranged or extending along a respective spherical surface, preferably with diameter increasing from the inner dome 10 to the outer dome 12 and intended to be mounted concentrically one over the other and so as to be able to mutually slide on one another concentrically.

The first dome or the inner dome 10 will be mounted on the main component 9 and will be pivoted or hinged to it, for example by a pin diametrically arranged along an axis z-z, preferably passing through the centre of the sphere itself and orthogonally to the longitudinal axis w-w. More particularly, such a pin is intended to be located, in use, on the front plane and in the inferior medial-lateral upper direction. The first dome 10 will thus be free to rotate only in the front and rear direction and can therefore measure the angle of anteversion of the femoral trial rasp or the implanted femoral prosthetic component. The measurement means may include graduated scales 9b imprinted on the main component 9 or on the first dome 10. Preferably, in its movement around the respective pin, the first dome 10 will drag with it the second 11 and third 12 dome too, since, preferably, the second dome 11 is integral with the first 10 and free to move only with respect to it, while the third dome 12 is, preferably, movable with the second and free to move only with respect thereto.

The second dome portion 11 will, preferably, be mounted on the first and hinged to this by the same pin between the first dome portion 10 and the main component 9, about the axis z-z, therefore also the second dome portion 11 can rotate only in the front and rear direction with respect to the first dome 10 and via the same it will therefore be possible to measure the angle of anteversion of the acetabulum and/or cotyle. The degrees referring to this angle can be detected on a suitable graduated scale 10a printed on the first 10 or the second 11 dome, while the sum of the two angles of anteversion will be detectable on a special graduated scale printed, for example, on the central sphere. The second dome during its movement about the respective hinge, will drag with it the third dome 12 too.

The third dome portion 12 will instead be mounted on the second 11 and hinged to this with one or more pins arranged diametrically and passing, in use, through the centre of rotation of the sphere and orthogonally to the longitudinal axis w-w on the sagittal plane and in the antero-posterior direction. The third dome will therefore preferably be free to rotate only in the lateral and the medial direction and orthogonally with respect to the first 10 and second 11 dome and can therefore measure the acetabular and/or cotyloid angle of inclination. The degrees of this angle can be detected on a suitable graduated scale printed indifferently on the second or the first dome or on the sphere.

The third dome 12 can then be provided with a first portion, for example, internal and a second portion, for example external that can be moved apart from/closer to the first portion, so as to increase or decrease the width of the auxiliary component to fit to cotyles of different sizes. To this regard, the third dome 12 may have a circular flange provided with a system of fins or flange portions which will expand as a result of actuation by suitable actuating means. These fins or flange portions by expanding will assume increasing diameters until coming into contact with the acetabular bone edge. This will be the ideal position of the prosthetic cotyloid component and will therefore be marked, for example with an electric scalpel, in order to be subsequently repeated.

Preferably, the auxiliary component/s 10, 11, 12 is/are designed to be located, in use, inside the or at the cotyle of a patient and, more preferably, the main component 9 too is designed to be located, in use, inside or at the cotyle of a patient.

The device 8 could also be provided or placed in electrical communication with infrared or telemetry means, data capture systems, robotised or computerised devices, etc.

The actuations and displacements described above, both with reference to the device 1 and to the device 8, may be obtained by any suitable system, such as a rack and pinion system, a worm screws and rack system, by means of simple or desmodromic cams so as to ensure, if necessary, the synchronisation of movements. Alternatively, the displacements could be controlled by means of a special key or brought to the outside of the surgical site with flexible cables or cardan shafts and actuated by means of sliders and or knobs on which the values of the different positions could also be read. Naturally, other means could also be used, mechanical, hydraulic, electrical, magnetic, electronic or of another nature.

The inventions according to the present application are applicable to any prosthetic hip system, of any manufacturing firm and/or distribution commercial firm.

The inventions are furthermore applicable both for prosthetic systems that provide femoral components, monobloc, with modular joint heads, monolithic stems or femoral stems with modular necks.

The invention is applicable to any surgical technique, surgical procedure, way of access, traditional techniques, techniques involving the use of navigators or robotised techniques, separately or in conjunction.

The invention relates to device 8 and a kit including the devices 1 and 8.

The measurements of the angles in the devices according to the present invention can be performed with references, goniometers, or other suitable systems.

The devices according to the present invention can be used both to make measurements and addressing intraoperative and laboratory choices, and to make measurements and addressing operations during working of implant sites, as well as to carry out inspection and measurement of prosthetic implants.

The devices according to the present invention can be implemented in either a disposable, or a "reusable" version, in which case the two components will be sterilised after each use.

A device 1 for a kit enables to perform a single joint reduction manoeuvre, since the values of the neck-shaft angle, of the standard offset or high offset, of the length of the prosthetic neck and of the lengths of the joint heads, of the ante- or retroversion of said neck can be varied with a reduced joint, if desired, by actuating on the trial neck, with a suitable key, a system or group of levers designed to change the mutual position of the components of the devices and hence, all the parameters, thus replicating all the different options available of the femoral prosthetic system in use.

Alternatively, the device can be structured in such a way that individual actuations are provided for each single configuration, with a second or other actuation intended to reproduce the different lengths of the joint head, so that, with extreme ease, accuracy and speed, the surgeon is able to explore all the possible solutions and choose the final configuration that best meets the required parameters.

In view of this, surgical intervention times are reduced as well as exposure of the part to be treated, which implies a reduction of the risk of surgical site infection and surgical joint damage, since several reduction and dislocation manoeuvres are avoided, resulting in greater minimal invasiveness, greater agility, safety and easier operation by the surgeon, in terms of choice of the final available configuration.

A device according to the present invention also allows the surgeon to operate using the so called "femur first" technique, which differs from standard techniques, in which first the cotyloid component is positioned, which is usually more susceptible to positioning errors, due to wear and possible arthrosis, and then, in a second phase, the femoral component, to then search for angular orientations of the cotyloid component, with a not ideal final result since a number of compromises must be accepted.

By contrast, according to the femur first technique, the femoral component is positioned first, and then, in a second phase, operation is carried out on the acetabulum, so as to position the cotyloid component with a suitable angular orientation, to position the device within the so-called "safe zone "; this orientation being obtained through the combination of anteversion of the femoral component and anteversion and inclination of the acetabular component, the joint is led to have an optimum range of motion as well as the best possible stability in a reduced condition, if not the complete elimination of the risk of post-surgical dislocations.

The difficulty in adopting the "femur first" technique faced with conventional devices is due to the practical impossibility to correctly and suitably measure, in the surgical field, the angular ratios between the femoral component and the cotyloid component before having carried out the definitive work, if not through the adoption and use of navigation techniques and or through robotised surgery techniques; such techniques, however, imply very high costs, longer surgical times, as well as the intervention of highly trained surgeons.

The invention solves these problems by ensuring an extremely easy, accurate and effective system, since it is already applicable to the initiating femoral rasp or the first rasp (since the operation is carried out by using several rasps of increasing size, and the orientations can be modified between the first and the last final rasp), thus enabling, even before the final preparation of the still adjustable femoral component, to know and accurately measure both the final configuration of the final femoral component and the angular ratios suitable and functional for the "safe zone" of the cotyloid component, enabling to choose the most suitable configuration of the prosthetic cotyloid component and or its insert, of the shoulder type or not, any protrusion, inclination, eccentric, etc., allowing the marking of suitable landmarks on the acetabular bone for the subsequent operation and implanting of the acetabular or cotyloid component.

This is achieved, as aforesaid, by mounting a device 8 on the adjustable trial neck, which device does not alter the final adoptable configuration, since it is constantly centred with respect to the centre of rotation of the joint according to the configuration in question.

This device enables the advancement and the completion of the operations with certainty of the final obtained result, allowing, however, for intermediate and final inspection both with the trial components, such as femoral stem and cotyle, and with the final implant components.

In short, the invention consists of a device and a kit provided with two devices, one relating to the intraoperative choice of the various available configurations of the femoral component and enables to treat the patient with a single reduction and dislocation manoeuvre and can be used in all surgical techniques, while the second is intended to measure the angular ratios between the femoral prosthetic component and the cotyloid prosthetic component and can be used as an aid and guide in the intraoperative choice of the versions of the femoral prosthetic component in relation to the attainment of the "safe zone" and or as a measuring, operation and placement guide of the cotyloid prosthetic component in the "femur first" technique.

The invention can be built to adapt to all the existing configurations of all the existing prosthetic femoral systems as it is composed of elements that are articulated and move one with respect to the other, so as to reproduce the various positions of the joint centre of rotation provided by each system.

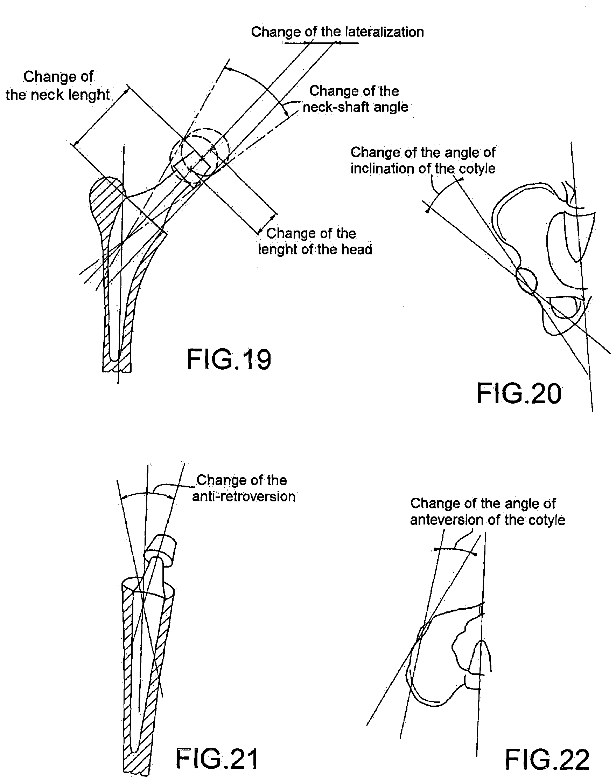

In the Figures 19 to 22 the measurable angles have been schematically shown with the devices according to the present invention.

The devices according to the present invention can be made disposable or reusable and can have control means, measurement means and data detection means of any type.

Modifications and variations of the invention are possible within the scope of the appended claims.

## Claims

1. Device for measuring the angle of anteversion of the femoral component, the angle of anteversion of the acetabulum and/or of the cotyle and/or the acetabular inclination angle and/or the cotyloid inclination angle for making a hip prosthesis, comprising:
- at least one main component (9) mountable on an apical component (3, 30) of a trial neck device or on an implanted femoral prosthesis component; and
- engagement means of said main component (9) with said apical component (3, 30) or with said femoral prosthesis component along a longitudinal axis (w-w), **characterised in that** the device comprises at least one auxiliary component (10, 11, 12) engageable with said main component (9), said at least one auxiliary component (10, 11, 12) being displaceable with respect to said main component (9) about an axis transversal to said longitudinal axis (w-w),
wherein said at least one auxiliary component (10, 11, 12) is pivoted or hinged to said at least one main component (9).

2. Device according to claim 1, **characterised in that** it comprises means for measuring the displacement of said at least one auxiliary component (10, 11, 12) with respect to said main component (9).

3. Device according to claim 1 or 2, **characterised in that** said transversal axis is substantially orthogonal to said longitudinal axis (w-w).

4. Device according to claim 3, **characterised in that** said transversal axis is arranged:
- on the front plane and in the inferior medial-lateral upper direction, so that the displacement of said auxiliary component with respect to said main component (9) allows the angle of anteversion of the femoral trial rasp or of the implanted femoral prosthesis component or the angle of anteversion of the acetabulum and/or of the cotyle to be measured; and/or
- on the sagittal plane and in the antero-posterior direction, so that the displacement of said auxiliary component with respect to said main component (9) allows the acetabular and/or cotyloid angle of inclination to be measured.

5. Device according to any one of claims 1 to 4, **characterised in that** said main component (9) comprises a substantially curved outer surface.

6. Device according to claim 5, **characterised in that** said substantially curved outer surface is at least partially sphere-shaped.

7. Device according to claim 5 or 6, **characterised in that** said auxiliary component (10, 11, 12) is shell-shaped or dome-shaped mountable on and at least partially around said main component (9).

8. Device according to claim 7, **characterised in that** it comprises two or more dome-shaped components (10, 11 and 12) designed to be mounted concentrically one over the other and so as to be able to mutually slide on one another concentrically.

9. Device according to claims 5 and 8, wherein said two or more dome-shaped components (10, 11, 12) comprise three auxiliary portions (10, 11, 12) configured as a shell or dome, i.e. a first dome or inner dome (10), a second dome portion (11) and a third dome portion (12) with diameter increasing from the inner dome (10) to the third dome portion (12) and intended to be mounted concentrically one over the other and so as to be able to mutually slide on one another concentrically.

10. Device according to claim 9, wherein said first dome or inner dome (10) is mounted on said main component (9) and is pivoted or hinged to it by a pin diametrically arranged along an axis (z-z) passing through the centre of the sphere and orthogonally to said longitudinal axis (w-w), such a pin being intended to be located, in use, on the front plane and in the inferior medial-lateral upper direction.

11. Device according to claim 9 or 10, wherein said second dome portion (11) is mounted on the first dome (10) and is hinged thereto by the same pin between said first dome (10) and said main component (9) about said axis (z-z) passing through the centre of said sphere, therefore the second dome portion (11) can rotate only in the front and rear direction with respect to the first dome (10) and via the same it is therefore possible to measure the angle of anteversion of the acetabulum and/or cotyle.

12. Device according to claim 9, 10 or 11, wherein said third dome portion (12) is mounted on the second dome (11) and hinged to this with one or more pins arranged diametrically and passing, in use, through the centre of rotation of the sphere and orthogonally to the longitudinal axis (w-w) on the sagittal plane and in the antero-posterior direction.

13. Device according to any previous claim, **characterised in that** said auxiliary component comprises at least one internal portion and one external portion movable apart from/closer to said inner portion, so as to increase or decrease the width of said auxiliary component to fit to cotyles of different sizes.

14. Device according to any previous claim, **characterized in that** said auxiliary component (10, 11, 12) is designed to be located inside or at the cotyle of a patient.

15. Device as claimed in claim 14, **characterized in that** said main component (9) is designed to be located inside or at the cotyle of a patient.

16. Kit for making a hip prosthesis, comprising a measuring device (8) according to any previous claim, as well as a trial neck device including at least one base component (2, 20, 200) and at least one apical or terminal component (3, 30) connected to said at least one base component (2, 20, 200), said at least one base component (2, 20, 200) being designed to be connected to a trial rasp (TR) or to a permanent prosthetic component, whereas said at least one apical component (3, 30) being designed to be arranged proximal to the cotyle, said trial neck device comprising means for adjusting the distance and/or the angular position of said at least one apical component (3, 30) with respect to said at least one base component (2, 20, 200), and wherein said at least one main component (9) is mountable on said apical component (3, 30) of said trial neck device.

17. Kit as claimed in claim 16, wherein said apical component (3) is configured as a cone or truncated cone, with a tapered cross section in the direction moving-away from the base component (2), and wherein said engagement means of said main component (9) with the apical component (3, 30) defines a housing seat (9a) of the apical component (3, 30) extending about a longitudinal axis (w-w).

## Patentansprüche

1. Vorrichtung zur Messung des Anteversionswinkels der Femurkomponente, des Anteversionswinkels der Hüftgelenkpfanne und/oder der Gelenkpfanne und/oder des Neigungswinkels der Hüftgelenkpfanne und/oder des Neigungswinkels der Gelenkpfanne zur Herstellung einer Hüftprothese, umfassend:
- mindestens eine Hauptkomponente (9), die an einer apikalen Komponente (3, 30) einer Probe-Halsvorrichtung oder an einer implantierten Femurprothesenkomponente montierbar ist; und
- Eingriffsmittel der besagten Hauptkomponente (9) mit der besagten apikalen Komponente (3, 30) oder mit der besagten Femurprothesenkomponente entlang einer Längsachse (w-w), **dadurch gekennzeichnet, dass** die Vorrichtung mindestens eine Hilfskomponente (10, 11, 12) umfasst, die mit der besagten Hauptkomponente (9) in Eingriff bringbar ist, die besagte mindestens eine Hilfskomponente (10, 11, 12) bezüglich der besagten Hauptkomponente (9) um eine Achse quer zu der besagten Längsachse (w-w) verschiebbar ist, wobei die besagte mindestens eine Hilfskomponente (10, 11, 12) an der besagten mindestens einen Hauptkomponente (9) schwenkbar oder angelenkt ist.

2. Vorrichtung nach Anspruch 1, **dadurch gekennzeichnet, dass** sie Mittel zum Messen der Verschiebung der besagten mindestens einen Hilfskomponente (10, 11, 12) in Bezug auf die besagte Hauptkomponente (9) umfasst.

3. Vorrichtung nach Anspruch 1 oder 2, **dadurch gekennzeichnet, dass** die besagte Querachse im Wesentlichen orthogonal zu der besagten Längsachse (w-w) ist.

4. Vorrichtung nach Anspruch 3, **dadurch gekennzeichnet, dass** die besagte Querachse angeordnet ist:
- auf der Frontebene und in der unteren medial-lateralen oberen Richtung, sodass die Verschiebung der besagten Hilfskomponente in Bezug auf die besagte Hauptkomponente (9) die Messung des Anteversionswinkels der Femurprobe-Raspel oder der implantierten Femurprothesenkomponente oder des Anteversionswinkels der Hüftgelenkpfanne und/oder der Gelenkpfanne ermöglicht; und/oder
- auf der Sagittalebene und in antero-posteriorer Richtung, sodass die Verschiebung der besagten Hilfskomponente in Bezug auf die besagte Hauptkomponente (9) die Messung des Neigungswinkels der Hüftgelenkpfanne und/oder der Gelenkpfanne ermöglicht.

5. Vorrichtung nach irgendeinem der Ansprüche 1 bis 4, **dadurch gekennzeichnet, dass** die besagte Hauptkomponente (9) eine im Wesentlichen gekrümmte Außenfläche aufweist.

6. Vorrichtung nach Anspruch 5, **dadurch gekennzeichnet, dass** die im Wesentlichen gekrümmte Außenfläche mindestens teilweise kugelförmig ist.

7. Vorrichtung nach Anspruch 5 oder 6, **dadurch gekennzeichnet, dass** die besagte Hilfskomponente (10, 11, 12) schalen- oder kuppelförmig auf dem und mindestens teilweise um die besagte Hauptkomponente (9) herum montierbar ist.

8. Vorrichtung nach Anspruch 7, **dadurch gekennzeichnet, dass** sie zwei oder mehr kuppelförmige Komponenten (10, 11 und 12) umfasst, die ausgebildet sind, um konzentrisch übereinander angebracht zu werden und so, dass sie gegenseitig konzentrisch aufeinander gleiten können.

9. Vorrichtung nach Anspruch 5 und 8, worin die besagten zwei oder mehr kuppelförmigen Komponenten (10, 11, 12) drei Hilfsabschnitte (10, 11, 12) umfassen, die als Schale oder Kuppel ausgebildet sind, d.h. eine erste Kuppel oder innere Kuppel (10), einen zweiten Kuppelabschnitt (11) und einen dritten Kuppelabschnitt (12) mit einem Durchmesser, der von der inneren Kuppel (10) zu dem dritten Kuppelabschnitt (12) zunimmt und dazu bestimmt ist, konzentrisch übereinander angebracht zu werden und so, dass sie gegenseitig konzentrisch aufeinander gleiten können.

10. Vorrichtung nach Anspruch 9, worin die besagte erste Kuppel oder innere Kuppel (10) auf der besagten Hauptkomponente (9) angebracht ist und schwenkbar oder angelenkt daran ist durch einen Stift, der diametral entlang einer durch das Zentrum der Kugel verlaufenden Achse (z-z) und orthogonal zu der Längsachse (w-w) angeordnet ist, wobei ein solcher Stift dazu bestimmt ist, bei Verwendung auf der Frontebene und in der unteren medial-lateralen oberen Richtung angeordnet zu sein.

11. Vorrichtung nach Anspruch 9 oder 10, worin der besagte zweite Kuppelabschnitt (11) an der ersten Kuppel (10) angebracht ist und mit demselben Stift zwischen der besagten ersten Kuppel (10) und der besagten Hauptkomponente (9) um die besagte Achse (z-z) angelenkt ist, die durch das Zentrum der besagten Kugel verläuft, sodass der besagte zweite Kuppelabschnitt (11) nur in die Vorwärts- und Rückwärtsrichtung in Bezug auf die erste Kuppel (10) drehen kann und es über denselben daher möglich ist, den Anteversionswinkel des Hüftgelenkpfanne und/oder der Gelenkpfanne zu messen.

12. Vorrichtung nach Anspruch 9, 10 oder 11, worin der besagte dritte Kuppelabschnitt (12) an der besagten zweiten Kuppel (11) angebracht und an dieser mit einem Stift oder mehreren Stiften, die diametral und, bei Verwendung, durch das Drehzentrum der Kugel verlaufend und orthogonal zur Längsachse (w-w) auf der Sagittalebene und in antero-posteriorer Richtung angeordnet sind, angelenkt ist.

13. Vorrichtung nach irgendeinem vorhergehenden Anspruch, **dadurch gekennzeichnet, dass** die besagte Hilfskomponente mindestens einen inneren Abschnitt und einen äußeren Abschnitt umfasst, der von dem besagten inneren Abschnitt wegbewegt/diesem genähert werden kann, um die Breite der besagten Hilfskomponente zu vergrößern oder zu verkleinern, sodass sie sich den verschiedenen Größen der Gelenkpfanne anpasst.

14. Vorrichtung nach irgendeinem vorhergehenden Anspruch, **dadurch gekennzeichnet, dass** die besagte Hilfskomponente (10, 11, 12) gestaltet ist, um innerhalb oder an der Gelenkpfanne eines Patienten angeordnet zu werden.

15. Vorrichtung nach Anspruch 14, **dadurch gekennzeichnet, dass** die besagte Hauptkomponente (9) gestaltet ist, um innerhalb oder an der Gelenkpfanne eines Patienten angeordnet zu werden.

16. Kit zur Herstellung einer Hüftprothese, umfassend eine Messvorrichtung (8) nach irgendeinem vorhergehenden Anspruch sowie eine Probehals-Vorrichtung mit mindestens einer Basiskomponente (2, 20, 200) und mindestens einer apikalen oder Abschlusskomponente (3, 30), die mit der besagten mindestens einen Basiskomponente (2, 20, 200) verbunden ist, wobei die besagte mindestens eine Basiskomponente (2, 20, 200) zur Verbindung mit einer Probe-Raspel (TR) oder mit einer permanenten prothetischen Komponente ausgelegt ist, während die besagte mindestens eine apikale Komponente (3, 30) ausgebildet ist, um proximal zur Gelenkpfanne angeordnet zu werden, wobei die besagte Probehals-Vorrichtung Mittel zum Einstellen des Abstands und/oder der Winkelposition der besagten mindestens einen apikalen Komponente (3, 30) in Bezug auf die besagte mindestens eine Basiskomponente (2, 20, 200) umfasst und worin die besagte mindestens eine Hauptkomponente (9) auf der besagten apikalen Komponente (3, 30) der besagten Probehals-Vorrichtung montierbar ist.

17. Kit nach Anspruch 16, worin die besagte apikale Komponente (3) als ein Kegel oder Kegelstumpf ausgebildet ist, mit einem verjüngten Querschnitt in die Richtung, die sich von der Basiskomponente (2) weg erstreckt, und worin die besagten Eingriffmittel der besagten Hauptkomponente (9) mit der apikalen Komponente (3, 30) einen Gehäusesitz (9a) der apikalen Komponente (3, 30) definieren, der sich um eine Längsachse (w-w) erstreckt.

## Revendications

1. Dispositif pour mesurer l'angle d'antéversion du composant fémoral, l'angle d'antéversion de l'acétabulum et/ou du cotyle et/ou l'angle d'inclinaison acétabulaire et/ou l'angle d'inclinaison cotyloïde pour réaliser une prothèse de hanche, comprenant :
- au moins un composant principal (9) montable sur un composant apical (3, 30) d'un dispositif d'essai pour le col ou sur un composant implanté de prothèse fémorale ;
- moyens d'engagement dudit composant principal (9) avec ledit composant apical (3, 30) ou avec ledit composant de prothèse fémorale le long d'un axe longitudinal (w-w), **caractérisé en ce que** le dispositif comprend au moins un composant auxiliaire (10, 11, 12) pouvant être engagé avec ledit composant principal (9), ledit au moins un composant auxiliaire (10, 11, 12) étant déplaçable par rapport audit composant principal (9) autour d'un axe transversal audit axe longitudinal (w-w),
dans lequel ledit au moins un composant auxiliaire (10, 11, 12) est articulé ou monté à charnière sur ledit au moins un composant principal (9).

2. Dispositif selon la revendication 1, **caractérisé en ce qu'**il comprend des moyens pour mesurer le déplacement dudit au moins un composant auxiliaire (10, 11, 12) par rapport audit composant principal (9).

3. Dispositif selon la revendication 1 ou 2, **caractérisé en ce que** ledit axe transversal est sensiblement orthogonal audit axe longitudinal (w-w).

4. Dispositif selon la revendication 3, **caractérisé en ce que** ledit axe transversal est disposé :
- sur le plan frontal et dans la direction inférieure médiale-latérale supérieure, de telle sorte que le déplacement dudit composant auxiliaire par rapport audit composant principal (9) permet de mesurer l'angle d'antéversion de la rugine d'essai fémoral ou du composant implanté de prothèse fémorale ou l'angle d'antéversion de l'acétabulum et/ou du cotyle; et/ou
- sur le plan sagittal et dans la direction antéro-postérieure, de telle sorte que le déplacement dudit composant auxiliaire par rapport audit composant principal (9) permette de mesurer l'angle d'inclinaison acétabulaire et/ou cotyloïde.

5. Dispositif selon l'une des revendications 1 à 4, **caractérisé en ce que** ledit composant principal (9) comprend une surface extérieure sensiblement incurvée.

6. Dispositif selon la revendication 5, **caractérisé en ce que** ladite surface extérieure sensiblement incurvée est au moins partiellement en forme sphérique.

7. Dispositif selon la revendication 5 ou 6, **caractérisé en ce que** ledit composant auxiliaire (10, 11, 12) est en forme de coquille ou en forme de dôme montable sur et au moins partiellement autour dudit composant principal (9).

8. Dispositif selon la revendication 7, **caractérisé en ce qu'**il comprend deux ou plusieurs composants en forme de dôme (10, 11 et 12) conçus pour être montés concentriquement l'un sur l'autre de façon à pouvoir coulisser mutuellement l'un sur l'autre concentriquement.

9. Dispositif selon les revendications 5 et 8, dans lequel lesdits deux ou plusieurs composants en forme de dôme (10, 11, 12) comprennent trois parties auxiliaires (10, 11, 12) configurées comme une coquille ou un dôme, c'est-à-dire un premier dôme ou dôme intérieur (10), une deuxième partie de dôme (11) et une troisième partie de dôme (12) dont le diamètre augmente depuis le dôme intérieur (10) jusqu'à la troisième partie de dôme (12) et destinés à être montés concentriquement l'un sur l'autre et de façon à pouvoir coulisser mutuellement l'un sur l'autre concentriquement.

10. Dispositif selon la revendication 9, dans lequel ledit premier dôme ou dôme intérieur (10) est monté sur ledit composant principal (9) et est articulé ou monté à charnière sur lequel avec une goupille disposée diamétralement le long d'un axe (z-z) traversant le centre de la sphère et orthogonalement audit axe longitudinal (w-w), ladite goupille étant destinée à être située, pendant l'utilisation, sur le plan frontal et dans la direction inférieure médiale-latérale supérieure.

11. Dispositif selon la revendication 9 ou 10, dans lequel ladite deuxième partie de dôme (11) est montée sur le premier dôme (10) et est montée à charnière sur celui-ci avec la même goupille entre ledit premier dôme (10) et ledit composant principal (9) autour dudit axe (z-z) traversant le centre de ladite sphère, par conséquent la deuxième partie de dôme (11) peut tourner seulement dans la direction frontale et arrière par rapport au premier dôme (10) et grâce à celle-ci il est donc possible de mesurer l'angle d'antéversion de l'acétabulum et/ou du cotyle.

12. Dispositif selon la revendication 9, 10 ou 11, dans lequel ladite troisième partie de dôme (12) est montée sur le deuxième dôme (11) et montée à charnière sur celui-ci avec une ou plusieurs goupilles disposées diamétralement et traversant, pendant l'utilisation, le centre de rotation de la sphère et orthogonalement à l'axe longitudinal (w-w) sur le plan sagittal et dans la direction antéro-postérieure.

13. Dispositif selon l'une quelconque des revendications précédentes, **caractérisé en ce que** ledit composant auxiliaire comprend au moins une partie interne et une partie externe mobile pour s'éloigner/se rapprocher de ladite partie intérieure, afin d'augmenter ou de diminuer la largeur dudit composant auxiliaire pour s'adapter aux cotyles de différentes tailles.

14. Dispositif selon l'une quelconque des revendications précédentes, **caractérisé en ce que** ledit composant auxiliaire (10, 11, 12) est conçu pour être situé à l'intérieur ou sur le cotyle d'un patient.

15. Dispositif selon la revendication 14, **caractérisé en ce que** ledit composant principal (9) est conçu pour être situé à l'intérieur ou sur le cotyle d'un patient.

16. Kit pour réaliser une prothèse de hanche, comprenant un dispositif de mesure (8) selon l'une quelconque des revendications précédentes, ainsi qu'un dispositif d'essai de col comprenant au moins un composant de base (2, 20, 200) et au moins un composant apical ou terminal (3, 30) raccordé audit au moins un composant de base (2, 20, 200), ledit au moins un composant de base (2, 20, 200) étant conçu pour être raccordé à une rugine d'essai (TR) ou à un composant prothétique permanent, alors que ledit au moins un composant apical (3, 30) est conçu pour être disposé près du cotyle, ledit dispositif d'essai de col comprenant des moyens pour ajuster la distance et/ou la position angulaire dudit au moins un composant apical (3, 30) par rapport audit au moins un composant de base (2, 20, 200), et dans lequel ledit au moins un composant principal (9) peut être monté sur ledit composant apical (3, 30) dudit dispositif d'essai de col.

17. Kit selon la revendication 16, dans lequel ledit composant apical (3) est configuré comme un cône ou un cône tronqué, avec une section transversale effilée dans la direction en éloignement du composant de base (2), et dans lequel lesdits moyens d'engagement dudit composant principal (9) avec le composant apical (3, 30) définissent un logement (9a) du composant apical (3, 30) s'étendant autour d'un axe longitudinal (w-w).
